(19) Europäisches Patentamt
European Patent Office
Office européen des brevets

(11) Veröffentlichungsnummer: **0 251 249**
**A2**

# (12) EUROPÄISCHE PATENTANMELDUNG

(21) Anmeldenummer: **87109236.7**

(22) Anmeldetag: **26.06.87**

(51) Int. Cl.⁴: **C07F 9/09** , B01F 17/38

(30) Priorität: **04.07.86 DE 3622440**

(43) Veröffentlichungstag der Anmeldung:
**07.01.88 Patentblatt 88/01**

(84) Benannte Vertragsstaaten:
**AT BE CH DE ES FR GB IT LI LU NL SE**

(71) Anmelder: **Henkel Kommanditgesellschaft auf Aktien**
**Postfach 1100 Henkelstrasse 67**
**D-4000 Düsseldorf-Holthausen(DE)**

(72) Erfinder: **Zeidler, Ulrich, Dr.**
**Heinrich-Lersch-Strasse 19**
**D-4000 Düsseldorf 13(DE)**
Erfinder: **Meffert, Alfred, Dr.**
**Marie-Curie-Strasse 10**
**D-4019 Monheim(DE)**

## (54) Alkyl-hydroxyalkyl-phosphorsäureester.

(57) Alkyl-hydroxyalkyl-orthophosphorsäureester-Gemische, bestehend aus Verbindungen der Formel I

$$\text{(I)} \qquad R^1 - O - \overset{\overset{\displaystyle O}{|}}{\underset{\underset{\displaystyle OR^3}{|}}{P}} - O - CH_2 - CH(OH)\ R^2$$

in der $R^1$ und $R^2$ Alkylgruppen mit 6 bis 22 C-Atomen und $R^3$ Wasserstoff, eine Gruppe $R^1$ oder eine Gruppe $-CH_2-CH(OH)-R^2$ ist, lassen sich herstellen durch Umsetzung von Phosphorpentoxid mit Fettalkohol und weitere Umsetzung des dabei gebildeten Phosphorsäurepartialester-Gemisches mit langkettigen Alpha-Epoxiden. Nach Überführung der Verbindungen der Formel I, in welchen $R^3$ = Wasserstoff ist, in die Salzform, eignen sich die neuen Alkylhydroxyalkyl-orthophosphorsäureester-Gemische als Emulgatoren.

EP 0 251 249 A2

## Alkyl-hydroxyalkyl-phosphorsäureester

Gegenstand der Erfindung sind neue Alkyl-hydroxyalkylorthophosphorsäure-Estergemische sowie ein Verfahren zu deren Herstellung.

Die Herstellung von o-Phosphorsäurepartialestern durch Umsetzung von Fettalkoholen mit Phosphorpentoxid und die Verwendung solcher Ester und deren wasserlöslicher Salze als oberflächenaktive Stoffe und insbesondere als kosmetische Emulgatoren ist seit langem bekannt. Es ist auch bekannt, Phosphorsäure mit langkettigen alpha-Epoxiden zu Phosphorsäure-2-hydroxyalkylestern, z. B. nach US-PS 3,487,130 umzusetzen.

Obwohl die Verwendung solcher Ester, insbesondere der wasserlöslichen Salze der Partialester als Emulgatoren für kosmetische und technische Emulsionen seit langem bekannt ist, wurde nunmehr überraschend gefunden, daß sich feinteiligere und stabilere Emulsionen mit Hilfe von Alkyl-hydroxyalkyl-phosphorsäureester-Gemischen herstellen lassen.

Gegenstand der Erfindung sind daher neue Alkyl-hydroxyalkylorthophosphorsäureester-Gemische, die dadurch gekennzeichnet sind, daß sie im wesentlichen aus Verbindungen der Formel I bestehen,

(I) 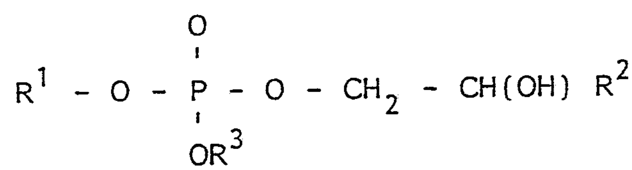

in der $R^1$ und $R^2$ Alkylgruppen mit 6 bis 22 C-Atomen und $R^3$ Wasserstoff, eine Gruppe $R^1$ oder eine Gruppe $-CH_2-CH(OH)-R^2$ ist. Besonders gute Emulgatoreigenschaften weisen solche erfindungsgemäßen Alkyl-hydroxyalkyl-orthophosphorsäureester-Gemische der Formel I auf, in welchen $R^1$ und $R^2$ lineare Alkylgruppen mit 10 bis 18 C-Atomen sind.

Die neuen Alkyl-hydroxyalkyl-orthophosphorsäureester-Gemische lassen sich nach einem sehr einfachen Verfahren herstellen. Dieses Verfahren, welches ein weiterer Erfindungsgegenstand ist, ist dadurch gekennzeichnet, daß man 1 Mol Phosphorpentoxid mit 3 Mol eines primären Fettalkohols der Formel $R^1$-OH oder mit 3-x Mol des Fettalkohols und x Mol Wasser umsetzt, wobei x einen Wert von 0 bis 1 annehmen kann und das dabei gebildete Orthophosphorsäurepartialester-Gemisch mit 0,5 bis 3 Mol, bevorzugt mit 0,5 bis 2 Mol eines Epoxids der Formel

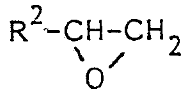

umsetzt.

$R^1$ und $R^2$ haben dabei die für Formel I angegebene Bedeutung. Die Umsetzung des Phosphorpentoxids mit Fettalkohol verläuft nach dem folgenden Formelschema

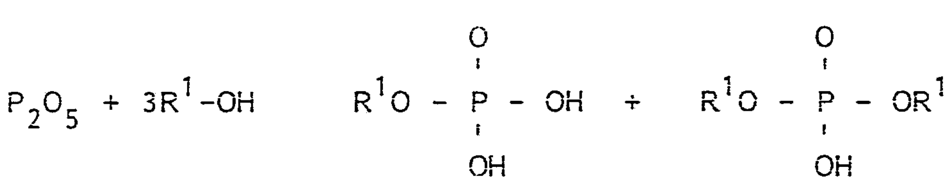

zu einem Gemisch aus Phosphorsäuremono-und diestern des Fettalkohols. Wird weniger als 3 Mol Fettalkohol pro Mol $P_2O_5$ eingesetzt, bilden sich Fettalkoholester der Pyrophophorsäure oder von Polyphosphorsäuren, je nach dem angewendeten Molverhältnis. In diesem Falle muß zur Spaltung der noch vorhandenen P-O-P-Bindungen Wasser zugesetzt werden. Es sollten aber nicht weniger als 2 Mol Fettalkohl pro Mol $P_2O_5$ zum Einsatz kommen, wenn man die erfindungsgemäßen Estergemische als Hauptprodukt erhalten will. Das auf diese Weise erhaltene Gemisch von Orthophosphorsäure-mono-und diestern des Fettalkohols wird dann mit 0,5 bis 3 Mol eines Alpha-Epoxids, bevorzugt eines linearen 1-Epoxyalkans der

Formel

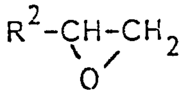

umgesetzt. Das Alpha-Epoxid wird bei dieser Reaktion teilweise oligomerisiert, so daß als Nebenprodukte im Estergemisch auch Phosphorsäureester enthalten sind, in welchen anstelle der Gruppe $-CH_2-CH(OH)R^2$ eine Gruppe $-(CH_2-CHR^2O)_xH$ steht, wobei x den Oligomerisationsgrad des Epoxids angibt. Auf diese Weise bleibt auch bei Einsatz von 3 Mol des Epoxids (pro Mol $P_2O_5$) noch ein Teil der P-OH-Gruppierungen erhalten, was sich in der Säurezahl des Phosphorsäureestergemisches zeigt.

Bevorzugt wird 1 Mol Phosphorpentoxid mit 3 Mol eines linearen primären Fettalkhols und das dabei gebildete Phosphorsäure-partialestergemisch mit 0,5 bis 2 Mol des Epoxids umgesetzt.

Die Umsetzung des $P_2O_5$ mit dem Fettalkohol ist exotherm, die Reaktionstemperatur sollte durch Kühlung in einem Bereich zwischen 70 und 100 °C gehalten werden. Auch die Umsetzung des Phosphorsäure-Fettalkohol-Partialesters mit dem Alpha-Epoxid ist exotherm und sollte durch Kühlung in einem Bereich zwischen 70 und 120 °C gehalten werden, damit hellfarbige Reaktionsprodukte erhalten werden.

Die erfindungsgemäßen Alkyl-hydroxyalkyl-orthophosphorsäureester-Gemische sind nach Maßgabe ihres Anteils an Verbindungen der Formel I, in der $R^3$ = Wasserstoff ist, durch eine mehr oder weniger große Säurezahl gekennzeichnet. Sie eignen sich nach Neutralisation mit Basen wie Alkalihydroxid oder Aminen zur Verwendung als oberflächenaktive Stoffe, insbesondere als Emulgatoren für technische und kosmetische Zwecke. Insbesondere eignen sie sich zur Überführung von kosmetischen Ölen und Fettstoffen in stabile, feinteilige Öl-in-Wasser-Emulsionen. Solche kosmetische Öl-und Fettkomponenten sind z. B. natürliche Öle vom Typ der ungesättigten Triglyceridöle wie z. B. Olivenöl, Sonnenblumenöl oder Mandelöl, Kohlenwasserstofföle wie z. B. Paraffinöl, flüssige Fettsäureester wie z. B. Hexyllaurat, Decyloleat, Isooctylstearat, Isopropylmyristat, Oleyloleat, synthetische Triglyceride wie z. B. Capryl-und Caprinsäure-triglycerid, Fettalkohole wie z. B. Oleylalkohol, synthetische verzweigte Alkohole wie z. B. 2-Octyldodecanol, 2-Hexyldecanol, Isostearylalkohol und viele andere als kosmetische Öl-und Fettkomponenten bekannte natürliche und synthetische Öle und Fette.

Bevorzugt werden die erfindungsgemäßen Alkyl-hydroxyalkyl-orthophosphorsäureester-Gemische nicht allein, sondern zusammen mit anderen, bekannten Emulgatoren und Konsistenzgebern verwendet. Bekannte Komponenten mit welchen die Alkyl-hydroxyalkyl-orthophosphorsäureester-Gemische gemeinsam verwendet werden, sind z. B. Fettsäurepartialglyceride, das sind Gemische von Glycerin-mono-und diestern von Fettsäuren mit 16 bis 22 C-Atomen und Cetyl-Stearylalkohol-Gemische. Zur Verwendung der Alkyl-hydroxyalkyl-orthophosphorsäureester-Gemische als Emulgatoren ist es erforderlich, die Verbindungen der Formel I, in welchen $R^3$ = Wasserstoff ist, in die Alkali-oder Ammoniumsalze oder die Salze anderer aliphatischer oder alicyclischer, primärer, sekundärer oder tertiärer Amine oder Alkanolamine mit 1 bis 12 C-Atomen, z. B. in die Mono-, Di-oder Trialkanolammoniumsalze mit 2 bis 4 C-Atomen in der Alkanolgruppe, in die Mono-, Di-oder Trialkylammoniumsalze mit 1 bis 4 C-Atomen in der Alkylgruppe oder in die Morpholiniumsalze zu überführen. Dies kann z. B. in der Weise geschehen, daß man durch Zugabe einer in Bezug auf die Säurezahl der Alkyl-hydroxyalkylphosphorsäureester-Gemische stöchiometrischer Mengen der Basen die Salze bildet und diese dann als Emulgatoren einsetzt. Man kann aber auch die sauren Alkyl-hydroxyalkyl-phosphorsäureester-Gemische der Ölphase zugeben und die Base zur wäßrigen Phase der Emulsion geben, so daß sich die als Emulgator wirksamen Salze erst während der Emulgierung bilden.

Die erfindungsgemäßen Alkyl-hydroxyalkyl-phosphorsäureester-Gemische werden zweckmäßigerweise in Mengen von 0,1 bis 10 Gew.-%, bezogen auf die gesamte Emulsion, eingesetzt. Bevorzugt werden sie in Mengen von 0,5 bis 5 Gew.-% zusammen mit 1 bis 25 Gew.-% (bezogen auf die Emulsion) an üblichen Co-Emulgatoren wie Fettsäurepartialglyceriden oder Cetyl-Stearylalkohol eingesetzt.

Die auf diese Weise erhältlichen Öl-in-Wasser-Emulsionen sind stabil, feinteilig und besonders glatt und glänzend und sehen daher besonders ansprechend aus. Sie eignen sich daher bevorzugt als kosmetische Hautpflegemittel.

Die folgenden Beispiele sollen den Gegenstand der Erfindung näher erläutern.

Beispiele

1. Herstellungsbeispiele

Allgemeine Herstellvorschrift

3 Mol eines $C_{12}/C_{14}$-Fettalkoholgemisches (Hydroxylzahl 290) wurden bei 20 °C in einem Reaktionsgefäß vorgelegt und 1 Mol Phosphorpentoxid nach und nach unter Rühren zugegeben. Nachdem die Reaktionstemperatur aufgrund der Reatktionswärme auf 80 °C gestiegen war, wurde diese Temperatur durch Kühlung während der weiteren Zugabe von $P_2O_5$ und noch weitere 2 Stunden beibehalten. Nach dieser Zeit hatte sich ein bei 20 °C halbfestes Veresterungsprodukt, bestehend aus einem Gemisch von Phosphorsäuremono-und Diester des Fettalkohols, gebildet, dessen Säurezahl 225 betrug.

Zu diesem Veresterungsprodukt wurden n Mol eines linearen Alpha-Epoxyalkans bei 50 °C zugetropft. Es setzte erneut eine exotherme Reaktion ein. Die Temperatur wurde durch Kühlung auf 90 °C gehalten, nach beenderter Zugabe wurde noch 2 Stunden bei 90 °C gerührt. Nach dieser Zeit war der Gehalt an Epoxidsauerstoff im Reaktionsgemisch auf 0 gesunken.

Es wurden wachsartige bis feste farblose bis schwach gelbe Produkte mit den aus der folgenden Tabelle zu entnehmenden Kennzahlen erhalten.

Tabelle

| Beispiel | 1,1 | 1,2 | 1,3 | 1,4 | 1,5 | 1,6 |
|---|---|---|---|---|---|---|
| $R^1OH$ | Lauryl-Myristylalkohol-Gemisch, OHZ: 290 | | | | | |
| $R^2$-CH-$CH_2$ \O/ | 1-Epoxy-dodecan/ 1-Epoxytetradecan-Gemisch, Epoxidsauerstoff: 7,65 Gew.-% | | | 1-Epoxyhexadecan/ 1-Epoxy-octadecan-Gemisch, Epoxidsauerstoff: 5,41 Gew.-% | | |
| n (Mol Epoxid pro Mol $P_2O_5$) | 1 | 0,6 | 1,4 | 1 | 0,6 | 1,4 |
| Säurezahl | 106,2 | 150,0 | 76,1 | 109,4 | 153,0 | 73,1 |
| Hydroxylzahl | 69,4 | 56,7 | 83,3 | 48,0 | 50,9 | 53,6 |
| Gew.-% P | 6,2 | 7,0 | 5,5 | 5,62 | 6,5 | 4,8 |

2. Prüfung der Emulgierwirkung

Die Emulgatoren gemäß 1,1 bis 1,6 wurden durch Zusatz einer der Säurezahl entsprechenden Menge N,N-Dimethylethanolamin (DMEA) in die N,N-Dimethylethanolammoniumsalze überführt. Die auf diese Weise erhaltenen DMEA-Salze wurden als Emulgatoren in die Testemulsionen gemäß Tabelle II eingesetzt. Die Herstellung der Emulsionen erfolgte wie üblich durch Vereinigung der Emulgatoren und Fettkomponenten bei einer Temperatur von ca. 70 °C, Zugabe der auf 75 °C erhitzten Wasserphase unter kräftigem Rühren und Abkühlen auf 20 °C nach Beendigung der Emulsionsbildung.

Die Emulsionen wurden 50 Tage bei -6 °C, + 23 °C und + 40 °C gelagert. Nach dieser Zeit war keine sichtbare Veränderung der Feinteiligkeit oder Stabilität der Emulsionen festzustellen.

Die Emulsionen waren gemäß Tabelle II zusammengesetzt:

Tabelle II

| Beispiel | 2,1 | 2,2 | 2,3 | 2,4 | 2,5 | 2,6 |
|---|---|---|---|---|---|---|
| DMEA-Salz von Beispiel 1,1 bis 1,6 | 3 | 0,7 | 3 | 1,5 | 1,5 | 4 |
| Cutina MD | 16 | 16 | - | 16 | 16 | 16 |
| Lanette O | - | - | 10 | - | - | - |
| Paraffinöl, dickflüssig | 6 | 6 | 5 | - | 6 | - |
| Eutanol G | - | - | 5 | - | - | 20 |
| Cetiol V | 6 | 6 | - | 10 | 6 | - |
| Mandelöl | - | - | - | - | - | 15 |
| Myritol 318 | 6 | 6,8 | - | 10 | 6,5 | - |
| Propylenglykol | - | - | - | - | - | 5 |
| Glycerin | - | - | - | 5 | - | - |
| Wasser | 63 | 64,5 | 77 | 57,5 | 64 | 40 |

In den Zusammensetzungen der Tabelle II wurden folgende Handelsnamen verwendet:

Cutina® MD: Gemisch von Mono-und Diglyceriden der Palmitin-und Stearinsäure (CTFA-Bezeichnung: Glyceryl-stearate)
Lanette ® O: Cetylstearylalkohol (CTFA-Bezeichnung: Cetearyl Alcohol)
Eutanol ® G: 2-Octyl-dodecanol
Cetiol V: Ölsäuredecylester (CTFA-Bezeichnung: Decyl Oleate)
Myritol (R)8 318: Capryl-/Caprinsäuretriglycerid (CTFA-Bezeichnung: Caprylic/Capric Triglyceride)
Es handelt sich um Handelsprodukte der Henkel KGaA, 4000 Düsseldorf (DE).

**Ansprüche**

1. Alkyl-hydroxyalkyl-orthophosphorsäureester-Gemisch, dadurch gekennzeichnet, daß es im wesentlichen aus Verbindungen der Formel I

$$(I) \qquad R^1-O-\overset{\overset{\displaystyle O}{|}}{\underset{\underset{\displaystyle OR^3}{|}}{P}}-O-CH_2-CH(OH)\,R^2$$

in der $R^1$ und $R^2$ Alkylgruppen mit 6 bis 22 C-Atomen und $R^3$ Wasserstoff, eine Gruppe $R^1$ oder eine Gruppe $-CH_2-CH(OH)-R^2$ ist, besteht.

2. Verfahren zur Herstellung eines Alkyl-hydroxyalkyl-phosphorsäureester-Gemisches nach den Ansprüchen 1 oder 2, dadurch gekennzeichnet, daß man 1 Mol Phosphorpentoxid mit 3 Mol Fettalkohol der Formel $R^1$-OH oder mit 3-x Mol Fettalkohol und x Mol Wasser umsetzt, wobei x einen Wert von 0 bis 1 annehmen kann und das dabei gebildete Phosphorsäurepartialester-Gemisch mit 0,5 bis 3 Mol eines Epoxids der Formel

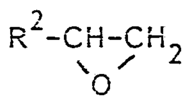

umsetzt.

3. Verfahren nach Anspruch 2, dadurch gekennzeichnet, daß man 1 Mol Phosphorpentoxid mit 3 Mol eines linearen primären Fettalkhols umsetzt und das dabei gebildete Phosphorsäurepartialester-Gemisch mit 0,5 bis 2 Mol des Epoxids umsetzt.

4. Verwendung von Alkyl-hydroxyalkylphosphorsäureester-Gemischen nach den Ansprüchen 1 oder 2 als Emulgatoren, dadurch gekennzeichnet, daß man die Verbindungen der For mel I, in welchen $R^3$ = Wasserstoff ist, in die Alkali-oder Ammoniumsalze oder die Salze anderer aliphatischer oder alicyclischer primärer, sekundärer oder tertiärer Amine oder Alkanolamine mit 1 bis 12 C-Atomen überführt.